# EUROPEAN PATENT SPECIFICATION

(11) **EP 0 424 117 B1**
(45) Date of publication and mention of the grant of the patent: **31.07.1996**
(21) Application number: 90311381.9
(22) Date of filing: 17.10.1990
(51) Int. Cl.: C12N 15/81, C12N 1/16, C12N 15/56, C12N 15/14

(54) **Yeast promoter**
Hefe-Promoter
Promoteur de levure

(30) Priority: 18.10.1989 GB 8923521
(43) Date of publication of application: 24.04.1991
(73) Proprietor: Delta Biotechnology Limited, Nottingham NG7 1FD (GB)
(72) Inventor: Goodey, Andrew Robert, Mapperley Park,Nottingham NG3 5DS (GB); Sleep, Darrell, West Bridgford,Nottingham NG2 5DS (GB); Vakeria, Dina, Chilwell,Nottingham NG9 4FZ (GB)
(74) Representative: Bassett, Richard Simon

(56) References cited:
- EP-A- 0 308 381
- JOURNAL OF BACTERIOLOGY, vol. 129, no. 3, March 1977, pages 1335-1342, American Society for Microbiology, US; G.F. SPRAGUE Jr., et al.: "Isolation and characterization of Saccharomyces cerevisiae mutants defective in glycerolcatabolism"
- CHEMICAL ABSTRACTS, vol. 109, no. 25, 19th December 1988, abstract no. 225504m, Columbus, Ohio, US; S.M. CHEN: & DISS. ABSTR: INT: B 1988, 49(3), 723

## Description

The present invention relates to yeast promoters, in other words nucleotide sequences which will direct expression of coding sequences in yeasts, for example Saccharomyces cerevisiae. Several such promoters have previously been isolated from yeasts and shown to be useful for directing the expression of heterologous coding sequences in yeast. The term "heterologous" in this specification is used to mean that the coding sequence is not the one whose expression is directed by the promoter in the wild-type organism in which the promoter is found; usually the coding sequence is one which is not found in the wild-type organism at all.

Sprague et al (1977 J. Bact. 129, 1335-1342) disclosed yeast mutants deficient in the GUT2 gene, which encodes a glycerol-3-phosphate dehydrogenase. Chen (1988 Diss. Abstracts Int. B 49(3), 723) disclosed the purification and characterisation of a yeast glycerol-3-phosphate dehydrogenase. Kingsman et al (1990 Methods in Enzymol. 185, 329-341) disclosed various promoters useful in yeast, including that of phosphoglycerate-kinase.

We have now found a further yeast promoter which can be used in this way, as can fragments of it, with advantages which were not predictable.

One aspect of the invention provides a DNA promoter sequence SEQ1, or a variant or a functional portion of said sequence, in isolation from the coding sequence which would normally neighbour the said sequence in wild-type Saccharomyces cerevisiae.

Although it will usually be undesirable, because a fusion protein will be produced when the promoter is used to express a heterologous protein, the promoter of the invention may be accompanied by a portion of the coding sequence which normally abuts it.

A "variant or functional portion" of the sequence is one which has minor variations of nucleotides and/or a shorter length, respectively, but which still retains at least 80% (preferably 90%, 95% or 99%) of the ability of the said sequence to promote transcription of a human albumin coding sequence positioned downstream thereof, with the other parameters of the two expression systems which are being compared (such as 3' regulatory regions) being the same. Alternatively, the variant or portion need retain only 10% of the said transcription-promoting activity provided that it is repressed by complex carbon sources and derepressed by the absence of such sources. In the case of a portion of the said sequence, such regulatory activity may be determined for the portion alone (ie without any other 5' regulatory sequence) or in conjunction with another 5' regulatory sequence positioned 5' or 3' to the said portion. A "variant" has 80%, preferably 90%, 95%, or 99% homology with the said sequence.

A "functional portion" of the sequence has 80%, or, preferably, 90%, 95%, 99% or 100% homology with the most homologous region of the said sequence. The portion is at least 100 nucleotides long, more preferably at least 200, 300, 400, 500, 1000 or 1500 nucleotides long. Suitably, the "functional portion" retains the ability of the said sequence to be repressed in the presence of complex carbon sources such as glucose and sucrose and to be derepressed in the absence of such sources whether or not glycerol or ethanol are present.

Suitably, the 3' end of any "functional portion" corresponds to the 3' end of SEQ1 and the said portion extends continuously away from the said 3' end in a 5' direction for up to about 1.35 or 1.40 kbp, beyond which (in the native environment) there appears to be a gene for Ala-tRNA^{GCU}. In nature, the 3' end of SEQ1 immediately precedes the ATG start codon.

Advantageously, the functional portion comprises SEQ3, in other words the 379 bp region immediately upstream of the ATG start codon, optionally with further 5' sections of the said sequence.

SEQ1, variants, portions and arrangements thereof described above are hereinafter referred to as a promoter of the invention.

The glycerol-3-phosphate dehydrogenase coding region has been shown to be homologous to the glycerol-3-phosphate dehydrogenase genes of mouse, rabbit, and Drosophila melanogasterwith which it has 64, 60 and 56 percent homology respectively. Glycerol-3-phosphate dehydrogenase is one of two enzymes required to convert glycerol into dihydroxyacetone phosphate; these are essential genes if glycerol is supplied as a sole carbon source. SEQ2 shows a part of the 5' region flanking the promoter and is constituted by the SEQ1 region plus 123 bp upstream thereof, and the ATG start codon.

A promoter of the invention may be located on a cloning vector or an expression vector adjacent a restriction site such that a heterologous coding sequence may be located downstream of the promoter and in correct reading frame in relation to a translational start codon. The start codon may be provided on the vector (eg immediately 3' to the promoter) or it may be inserted as a 5' end of the heterologous coding sequence. A linker may be provided between the promoter of the invention and the start codon, if desired. 3' regulatory regions may similarly be provided on the vector or inserted with the coding sequence. The transcription termination signal is preferably the 3' flanking sequence of a eukaryotic gene which contains proper signals for transcription termination and polyadenylation in fungi. Suitable 3' flanking sequences may, for example, be those of the GUT2 gene or they may be different. preferably the termination signal is that of the S. cerevisiae PGK1 orADH1 genes. Preferably, the DNAconstruct according to the present invention is provided at both ends with synthetic oligonucleotide linkers which allow insertion and cloning of the construct in a cloning vector. The promoter of the invention. the DNA coding sequence and the fungal transcription termination signals are operably linked to each other, ie they are juxtaposed in such a manner that their normal functions are maintained. Thus, the array is such that the expression control sequence effects proper expression of the coding sequence and the transcription termination signals effect proper termination of transcription and polyadenylation. The junction of these sequences is preferably effected by means of synthetic oligonucleotide linkers which may carry the recognition sequence of an endonuclease.

According to the present invention there is further provided a vector having one or multiple DNA inserts each comprising a promoter of the invention, a DNA segment consisting of a DNA sequence coding for a desired polypeptide which DNA segment is under transcriptional control of said promoter, and a DNA sequence containing eukaryotic transcription termination signals.

The vectors according to the invention are plasmids or linear DNA vectors and are selected depending on the host organism envisaged for transformation.

The invention relates also especially to plasmids which apart from the expression control sequence, the above DNA segment and the sequence containing transcription termination signals contain additional DNA sequences which are inessential or less important for the function of the promoter, ie for the expression of the desired polypeptide, but which perform important functions, for example in the propagation of the cells transformed with said plasmids. The additional DNA sequences may be derived from prokaryotic and/or eukaryotic cells and may include chromosomal and/or extra-chromosomal DNA sequences. For example, the additional DNA sequences may stem from (or consist of) plasmid DNA, such as bacterial or eukaryotic plasmid DNA, viral DNA and/or chromosomal DNA, such as bacterial, yeast or higher eukaryotic chromosomal DNA. Preferred plasmids contain additional DNA sequences derived from bacterial plasmids, especially Escherichia coli plasmid pBR322 or related plasmids, bacteriophage, yeast 2p plasmid, and/or yeast chromosomal DNA.

In the preferred plasmids according to the invention, the additional DNA sequences carry a yeast replication origin and a selective genetic marker for yeast. Plasmids containing a yeast replication origin, eg an autonomously replicating segment (ars), are extrachromosomally maintained within the yeast cells after transformation and are autonomously replicated upon mitosis. Plasmids containing sequences homologous to yeast 2p plasmid DNA can be used as well. These plasmids may be integrated by recombination into 2p plasmids already present within the cell or may replicate autonomously. The integration vectors of EP-A-251 744 or the "disintegration" vectors of EP-A-286 424 may be used.

As to the selective gene marker for yeast, any marker gene can be used which facilitates the selection for transformants due to the phenotypic expression of the marker. Suitable markers for yeast are particularly those expressing antibiotic resistance or, in the case of auxotrophic yeast mutants, genes which complement host lesions. Corresponding genes confer, for example, resistance to the antibiotic cycloheximide or provide for prototrophy in an auxotrophic yeast mutant, for example the URA1, URA3, ARG4, LEU2, HIS4, HIS3, TRP5 or TRP1 gene.

Advantageously, the additional DNA sequences which are present in the plasmids according to the invention also include a replication origin and a selective genetic marker for a bacterial host, especially Escherichia coli. There are useful features which are associated with the presence of an E. coli replication origin and an E. coli marker in a yeast hybrid plasmid. Firstly, large amounts of plasmid DNA can be obtained by growth and amplification in E. coli and, secondly, the construction of plasmids is conveniently done in E. coli making use of the whole repertoire of cloning technology based on E. coli. E. coli plasmids, such as pBR322 and the like, contain both E. coli replication origin and E. coli genetic markers conferring resistance to antibiotics, for example tetracycline and ampicillin, and are advantageously employed as part of the yeast vectors

The vectors according to the invention may contain one or multiple DNA inserts each comprising inter alia the expression control sequence and the DNA sequence encoding the desired protein. If the vectors contain multiple DNA inserts, for example 2 to 4 DNA inserts, these can be present in a tandem array or at different locations of the vector. Preferred vectors contain one DNA insert or DNA inserts in a tandem array. The DNA inserts are especially head to tail arranged.

The plasmids according to the invention are prepared by methods known in the art. The process for the preparation of the vectors comprises introducing one or multiple DNA constructs containing a promoter of the invention, a DNA segment consisting of a DNA sequence coding for a desired polypeptide which DNA segment is under transcriptional control of said expression control sequence, and a DNA sequence containing fungal transcription termination signals, as such or introducing the components of said DNA constructs successively in the predetermined order into a vector DNA.

The construction of the plasmids according tothe invention is performed applying conventional ligation techniques. The components of the plasmids are linked through common restriction sites and/or by means of synthetic linker molecules and/or by blunt end ligation.

A promoter of the invention may be used in transformed yeast, for example Saccharomyces cerevisiae or Schizosaccharomyces pombe, or in any other host in which the promoter is found to be effective. Fungal cells include the genera Pichia, Saccharomyces, Kluyveromyces, Candida, Torulopsis, Hansenula, Schizosaccharomyces, Citero- myces, Pachysolen, Debaromyces, Metschunikowia, Rhodosporidium, Leucosporidium, Botryoascus, Sporidiobolus, Endomycopsis, and the like. Preferred genera are those selected from the group consisting of Pickia, Saccharomyces, Kluyveromyces, Yarrowia and Hansenula, because the ability to manipulate the DNA of these yeasts has, at present, been more highly developed than for the other genera mentioned above. Examples of Saccharomyces are Saccharomyces cerevisiae, Saccharomyces italicus and Saccharomyces rouxii. Examples of Kluyveromyces are Kluyveromyces fragilis and Kluyveromyces lactis. Examples of Hansenula are Hansenula polymorpha, Hansenula anomala and Hansenula capsulata. Yarrowia lipolytica is an example of a suitable Yarrowia species. Filamentous fungi include Aspergillus niger.

Fungal cells can be transformed by: (a) digestion of the cell walls to produce spheroplasts; (b) mixing the spheroplasts with transforming DNA (derived from a variety of sources and containing both native and non-native DNA sequences); and (c) regenerating the transformed cells. The regenerated cells are then screened for the incorporation of the transforming DNA.

It has been demonstrated that fungal cells of the genera Pichia, Saccharomyces, Kluyveromyces, Yarrowia and Hansenula can be transformed by enzymatic digestion of the cells walls to give spheroplasts; the spheroplasts are then mixed with the transforming DNA and incubated in the presence of calcium ions and polyethylene glycol, then transformed spheroplasts are regenerated in regeneration medium.

Methods for the transformation of S. cerevisiae are taught generally in EP 251 744, EP 258 067 and WO 90/01063, all of which are incorporated herein by reference.

Alternatively, the transformation of yeast with the hybrid vectors may be accomplished according to the method described by Hinnen et al [Proc. Natl. Acad. Sci. USA 75, 1929 (1978)]. This method can be divided into three steps:
(1) Removal of the yeast cell wall or parts thereof using various preparations of glucosidases, such as snail gut juices (e.g. Glusulase^{R} or Helicase^{R}) or enzyme mixtures obtained from microorganisms (eg Zymolyase^{R}) in osmotically stabilized solutions (eg 1 M sorbitol).
(2) Treatment of the "naked" yeast cells (spheroplasts) with the DNA vector in the presence of PEG (polyethyleneglycol) and Ca2+ ions.
(3) Regeneration of the cell wall and selection of the transformed cells in a solid layer of agar. This regeneration is conveniently done by embedding the spheroplasts into agar. For example, molten agar (about 50°C) is mixed with the spheroplasts. Upon cooling the solution to yeast growth temperatures (about 30°C), a solid layer is obtained. This agar layer is to prevent rapid diffusion and loss of essential macromolecules from the spheroplasts and thereby facilitates regeneration of the cell wall. However, cell wall regeneration may also be obtained (although at lower efficiency) by plating the spheroplasts onto the surface of preformed agar layers.

Preferably, the regeneration agar is prepared in a way to allow regeneration and selection of transformed cells at the same time. Since yeast genes coding for enzymes of amino acid biosynthetic pathways are generally used as selective markers (-supra), the regeneration is preferably performed in yeast minimal medium agar. If very high efficiencies of regeneration are required the following two step procedure is advantageous:
(1) regeneration of the cell wall in a rich complex medium, and
(2) selection of the transformed cells by replica plating the cell layer onto selective agar plates.

When the DNAvector is a linear DNAvector used fortransforming eukaryotic host cells, transformation is preferably done in the presence of a second vector containing a selective marker for yeast. This cotransformation allows enrichment for those host cells which have taken up DNA that cannot be directly selected for. Since competent cells take up any type of DNA a high percentage of cells transformed with a selective vector will also harbour any additional DNA (such as the above linear DNAvector). The transformed host cells can be improved in production of the desired polypeptide by mutation and selection using methods known in the art. The mutation can be effected, for example, by U.V. irradiation or suitable chemical reagents. Strains which are deficient in protease A and B are particularly preferred; such strains are generally available.

The heterologous coding sequence may encode any desired polypeptide, including oligopeptides. The polypeptide may be fibronectin or a portion thereof (for example the collagen or fibrin-binding portions described in EP 207 751), urokinase, pro-urokinase, the 1-368 portion of CD4 (D Smith et al (1987) Science 328, 1704-1707) platelet derived growth factor (Collins et a/ (1985) Nature 316, 748-750), transforming growth factor β (Derynck et al (1985) Nature 316, 701-705), the 1-272 portion of Von Willebrand's Factor (Bontham et al, Nucl. Acids Res. 145 7125-7127), the Cathepsin D fragment of fibronectin (585-1578), α₁-antitrypsin, plasminogen activator inhibitors, factor VIII, a-globin, f-globin, myoglobin, nerve growth factor, LACI (lipoprotein-associated coagulation inhibitor) (Broze, G J. (1990) Biochem. 29, 7539-7546), lactoferrin (Fletcher, J. in "Iron in Immunity, Cancer & Inflammation" 1989, Wiley & Sons, Eds. de Sousa, M. & Brock, J. H.) or platelet-derived endothelial cell growth factor (PDECGF) (Ishikawa, F (1989) Nature 338, 557-562) or a conservative variant of any of these. The polypeptide may also be a fusion of HSA or an N-terminal portion thereof and any other polypeptide, such as those listed above. Preferably, the polypeptide is a naturally-occurring human serum albumin, a modified human serum albumin or a fragment of either, such modified forms and fragments being termed "variants", or is a- or b-globin. These variants include all forms or fragments of HSA which fulfill at least one of the physiological functions of HSA and which are sufficiently similar to HSA, in terms of structure (particularly tertiary structure) as to be regarded by the skilled man as forms or fragments of HSA.

In particular, variants or fragments of HSA which retain at least 50% of its ligand-binding properties (preferably 80%, or 95%), for example with respect to bilirubin or fatty acids, and/or at least 50% (preferably 80% or 90%) of its oncotic action are encompassed. Such properties are discussed in Brown, J R & Shockley, P (1982) in Lipid-Protein Interactions 1, 26-68, Ed. Jost, P C & Griffith, O H.

The portion of HSA disclosed in EP 322 094 is an example of a useful fragment of HSA which may be expressed by use of a promoter of the invention.

The polypeptide may initially be expressed as a fusion with a secretion leader sequence. In the case of HSA, this may for example, be the natural HSA leader the leader from the S. cerevisiae a mating factor, the Kluyveromyces lactis killer toxin leader or a fusion between the natural HSA leader and either of the said yeast leaders. Thus, the leader may be either of SEQ4 and SEQ5 or conservatively modified variations of either sequence, as described in WO 90/01063.

The host cell may be fermented to express the desired polypeptide in known ways. The polypeptide may be purified by known techniques, for example (if the polypeptide is not secreted) separating off the cells, lysing them, collecting the supernatant, concentrating it and chromatographically separating the polypeptide.

The promoter of the invention is de-repressed by the absence of complex carbon sources (whether or not glycerol and ethanol are present), which is advantageous in large scale yeast culture. Thus, the invention provides a process for growing the transformed yeast to a high mass and then inducing expression of the desired polypeptide by allowing the medium to become exhausted of complex carbon sources and adding a simpler carbon source such as glycerol or ethanol.

Preferred aspects of the invention will now be described by way of example and with reference to the accompanying drawings, in which:
Figures 1 to 9 are respective restriction maps of plasmids pXL5, pAYE274, pAYE275, pAYE334, pAYE276, pAYE323, pAYE324, pSAC35 and pAYE321;
Figure 10 is a photograph of a gel showing labelled RNA from a cell culture at differing times;
Figure 11 is a graph showing the time course of expression of the glycerol-3-phosphate dehydrogenase promoter corresponding to Figure 10;
Figure 12 is a restriction map of plasmid pDXL200;
Figure 13 is a restriction map of plasmid pDVX2; and
Figure 14 is a restriction map of plasmid pDVX4.

### Introduction

### Strains and Culture Conditions

Escherichia coli DH5α (F-, φ80dlacZdeltaM15,delta(lacZYA-argF) U169, recA1, endA1, hsdR17 (rₖ-, mₖ⁺), supE44, lambda, thi-1, gyrA, relA1) was used for plasmid constructions. E. coliXL1-blue (Stratagene, endA1, hsdR17 (rₖ-, mₖ⁺), supE44, thi-1, lambda, recA1, gyrA96, relA1,(lac-), [F', proAB, lad^{q} ZdeltaM15, Tn10, (tet^{r})] was used for the propagation of M13 vectors. Saccharomyces cerevisiae DB1 cir° (a, leu2) was used as the recombinant albumin expression host. Other S. cerevisiae strains used were AH22 cir⁺ (a, canl, leu2, his4); BJ1991 cir⁺ (a, prb1-1122, pep4-3, leu2, trpl, ura3-52) and S22 cir⁺ (a, ade1, ade2, ura1, his7, tyrl, lys7, gall, gut2). Yeast cells were grown at 30°C on YEP (1% (w/v) yeast extract, 2% (w/v) bactopeptone) nutrient agar supplemented with the appropriate carbon source S. cerevisiae transformants were grown in 10ml YEP, 2% (w/v) sucrose in 50ml conical shake flasks at 30°C, 200rpm for 72 hours. HSA antibody plates were prepared by cooling YEP containing 1% (w/v) electrophoresis grade agarose to 50°C. Rabbit anti-human albumin antiserum (Cambio, Cambridge, United Kingdom) was then added to 2.5% (v/v) along with the appropriate carbon source and the nutrient medium poured into a Petri dish and allowed to cool.

### DNA Manipulations

Standard DNA manipulation techniques were used (Maniatis etal 1982: Molecular Cloning, A Laboratory Manual; Cold Spring Harbor; Sambrook et a/1989 (2nd edition). DNA fragments were routinely recovered from agarose gels by centrifugation (Vogelstein, B., Anal. Biochem. 160 (1987) 115-118). Radiolabelled DNA was prepared using [a-³²P] dATP (Amersham International PLC) and the random primer labelling procedure (Feinburg, A P and Vogelstein, B., Anal. Biochem. 137, (1984) 266-267). Restriction endonucleases, T4 DNA ligase, T4 DNA Polymerase and E. coli DNA polymerase I (Klenow fragment) were obtained from Boehringer-Mannheim.

The glycerol-3-phosphate dehydrogenase yeast promoter fragment was obtained from a genomic library of fragments obtained by Bg/II restriction of yeast DNA. The Bg/II restriction fragments are inserted into a unique Bg/II site of a plasmid containing the Herpes Simplex thymidine kinase (TK) gene. Only when promoter fragments are cloned in front of the thymidine kinase gene will yeast transformed with this plasmid grow in the presence of folate antagonists such as sulphanilamide and amethopterin, as described by Goodey et al. Molecular and General Genetics 204, 505-511 (1986) which is incorporated herein by reference.

A plasmid having an active promoter was selected by measurement of thymidine kinase activity in the cell extract.

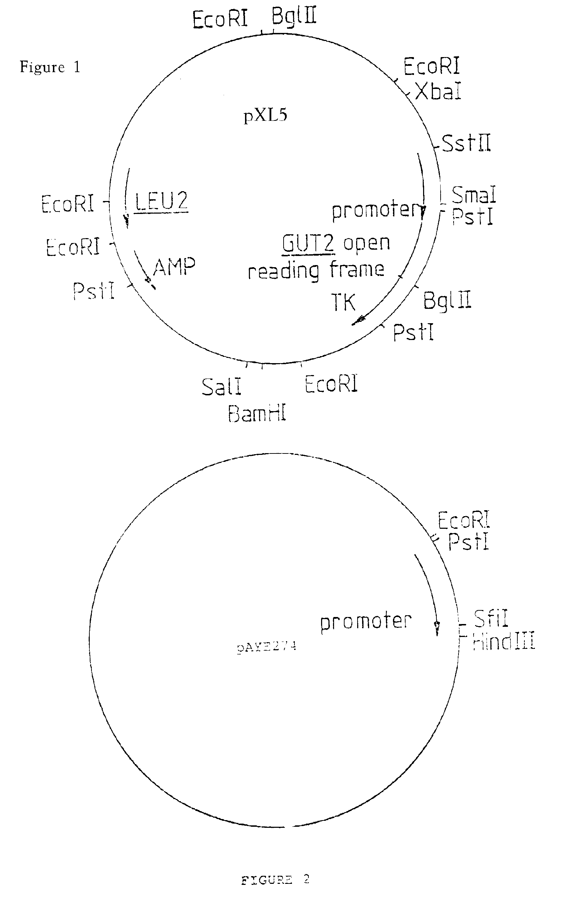

The promoter fragment was contained within a Bgnl restriction fragment of plasmid pXL5 (Figure 1). A 1.48kbp fragment of the glycerol-3-phosphate dehydrogenase promoter was sequenced (SEQ2). The promoter fragment was modified by the introduction of an SfiI restriction endonuclease site on the 3' end of the yeast promoter: These two sequences are SEQ6 and SEQ7 respectively.

### EXAMPLE I: Expression of recombinant Human Serum Albumin (rHA)

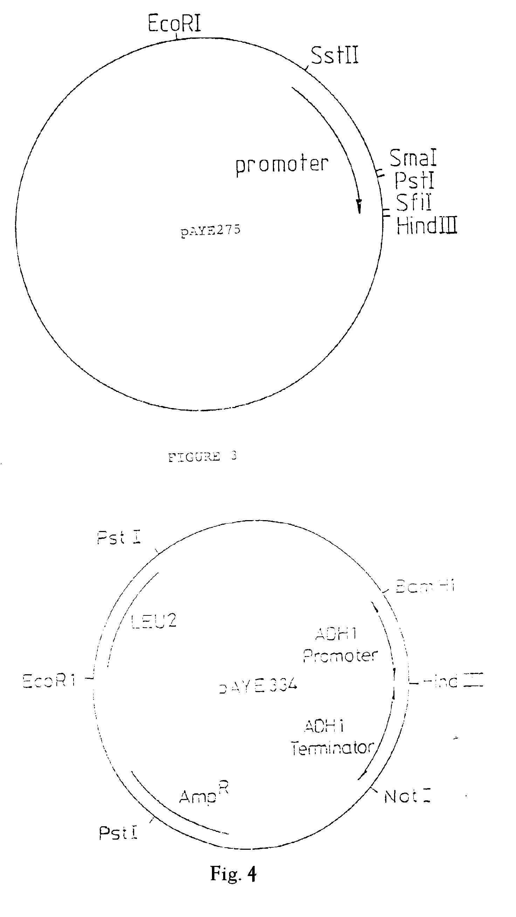

A 282bp Pstl-Rsal fragment of the promoter of the invention (ie from the CTGCAG at position 1031-1036 to the GTAC at 1314-1317 of SEQ1) and a 56bp double stranded oligonucleotide linker (the 5'-3' strand of which constitutes SEQ12) were inserted between the PstI and HindIII site of M13mp18 (Yanisch-Perron et al, 1985, Gene 33, 103-109) generating plasmid pAYE274 (Figure 2), so introducing a unique SfiI 5' to the translation initiation site. Plasmid pAYE274 was linearised with EcoRl and Pstl and recircularised with the 2.3kb EcoRl-Pstl fragment from pXL5 (Figure 1) generating pAYE275 (Figure 3). This was digested with EcoRl-Hindlll and the 2.3kb promoter fragment purified

The construction of plasmid pAYE334, which is used in the next stages of the work, has been described in our copending UK patent application No 8927480.7 but is repeated here.

Plasmid pAAH5 (Goodey etal. 1987: In Yeast Biotechnology, 401-429, Edited by Berry, D.R., Russell, I. and Stew- art, G.G. Published by Allen and Unwin) was linearised by partially digesting with BamHl. The 5' protruding ends were blunt-ended with T4 DNA polymerase and ligated with the double-stranded oligonucleotide linker:

A recombinant plasmid pAYE334 (Figure 4) was selected in which a Notl restriction site had replaced the BamHl site at the 3' end of the ADH1 terminator.

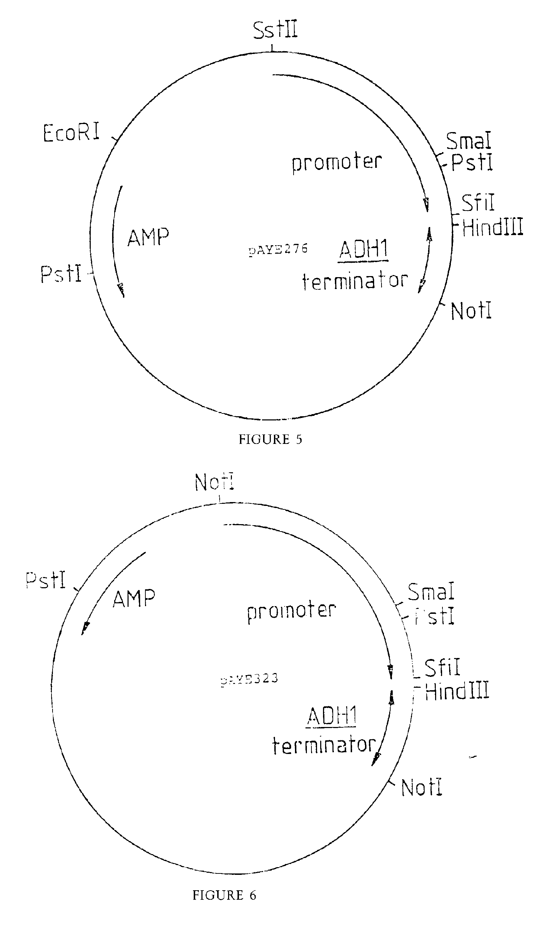

The modified promoter fragment from pAYE275 (Fig 3) and a 450bp HindIII-NotI ADH1 terminator fragment from pAYE334 were ligated into pAT153 (Twigg and Sherratt, 1980) which itself had been modified by the introduction of NotI recognition site (5'-GCGGCCGC-3') into and so destroying the BamHl site, generating pAYE276 (Figure 5).

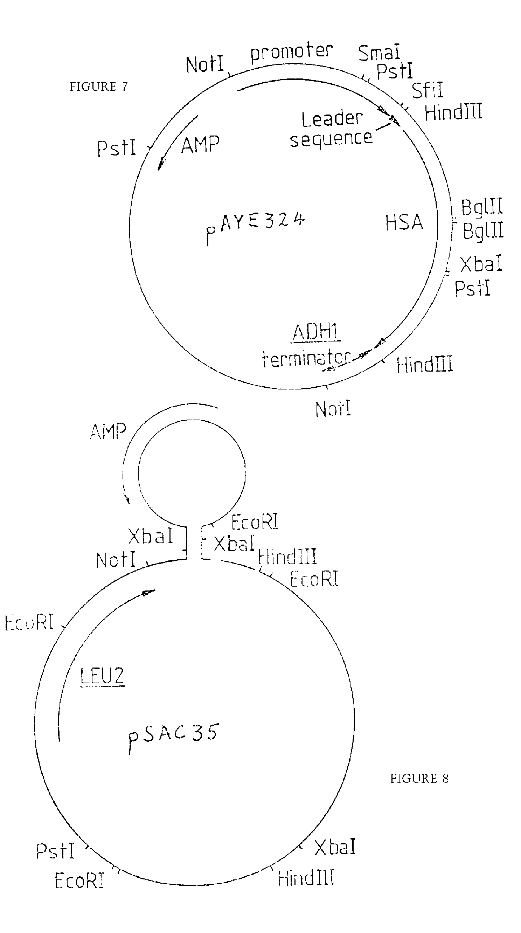

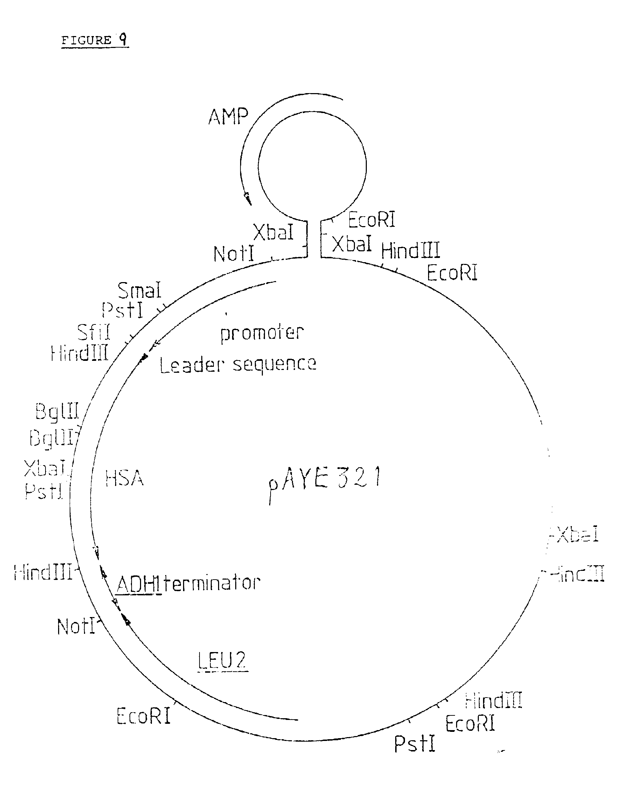

Plasmid pAYE276 was linearised with EcoRl-Sstll, the 3' recessed ends filled in the T4 DNA Polymerase and dNTP and recircularised with excess Notl linker (5'-GCGGCCGC-3') generating plasmid pAYE323 (Figure 6). This plasmid was linearised with Hindlll and recircularised with a double stranded oligonucleotide linker: (the 5'-3' strand of which constitutes SEQ13) and a 1.9kbp HA cDNA fragment liberated from Xhol linearised mp19.7 (EP-A-201 239), blunt ended with S1 nuclease and then digested with Hindlll, to create plasmid pAYE324 (Figure 7). The 3.72kbp Notl restriction fragment created in plasmid pAYE324 (Figure 7) may then be transferred into a suitable yeast replicating vector that contains a unique Notl restriction site (for example pSAC35, Figure 8), to create a plasmid such as pAYE321 (Figure 9).

Plasmid pSAC35 is a derivative of pSAC3 described by Chinery and Hinchliffe (1989) Curr. Genet. 16, 21-25, and in EP 286424. The LEU2 selectable marker is a 1.95 kbp Sa/I - Hpal fragment from YEP13, (Broach J R, et al (1979) Cell 16, 827-839) inserted into the SnaBl site of pSAC3. The LEU2 gene possesses a unique Tth111I site. Following digestion with this enzyme the 5' protruding ends were removed by treatment with the Klenow fragment of E. coli DNA Polymerase I. The insertion of a Notl recognition site to generate pSAC35 was achieved by ligating the blunt end linearised DNA with a double stranded oligonucleotide of the sequence, Those skilled in the art will recognise a large number of techniques for modifying DNA segments which code for a wide variety of proteins for insertion into an SfiI restriction site.

This Example describes an HSA secretion vector (pAYE321) incorporating a promoter of the invention. This vector has been used to transform five different yeast strains: all five strains secreted HSA into the culture supernatant. The timing of HSA expression under the control of the promoter has also been studied. HSA mRNA is first detected when the cells have reached late logarithmic growth. High levels of HSA mRNA are maintained even when the culture has entered stationary phase.

Plasmid pAYE324 (Figure 7) is a pAT153-based vector which possesses the entire promoter/HSA secretion cassette flanked by NotI restriction sites The 3.715kbp secretion cassette contains the following features:
i) A 1.35kbp promoter fragment which includes the native promoter ATG environment except that four nucleotide substitutions have been incorporated at a site between 30 and 40bp upstream of the ATG as described above (SEQ7). These substitutions introduce a unique SfiI restriction site in the 3' region of the promoter.
ii) The natural HSA/a-factor fusion leader sequence (WO 90/01063) directing the secretion of mature HSA.
iii) The yeast alcohol dehydrogenase ADH1 terminator region.

The 3.715kbp Nofl promoter/HSA secretion cassette was purified and inserted into the unique NotI cloning site of pSAC35 (Figure 8) to generate plasmid pAYE321 (Figure 9).

Five [cir°] strains were transformed to leucine prototrophy with plasmid pAYE321, namely Strain 1 [cir°], Strain 2 [cir°], Strain 3 [cir°], Strain 4 [cir°] and Strain 5 [cir°]. Transformation was performed essentially as described by Beggs (Nature, 275 (1978) 104-109) except for the following modifications. Transforming DNA in 10µl deionised H₂0 was gently mixed with 50µl of spheroplasts in 1.2M sorbitol, 10mM CaCl₂ and 12.5µl 20% (w/v) PEG 3350 (Sigma), 10mm CaCl₂, 10mM Tris/HCI (pH7.5) and held on ice for 15 minutes. After adding a further 500µl of 20% (w/v) PEG 3350, 10mM CaCl₂, 10mM Tris/HCI (pH7.5) the spheroplasts were gently mixed with 5ml of 1.2M sorbitol selective agar medium and plated out. Two independent transformants from each strain were grown for 72 hours, 200 rpm shaking, at 30°C in 10ml of YEP (1 % w/v yeast extract, 2% w/v bactopeptone and 2% w/v glucose).

HSA was detected in the culture supernatants of all the transformants, showing that the promoter can direct the expression/secretion of heterologous proteins in yeast.

### EXAMPLE 2: Timing of Expression

A one-litre shake flask containing 400ml of YEP, 2% (w/v) glucose was inoculated with Strain 1 pAYE321 and incubated at 30°C, 200 rpm Samples (20ml) were removed at 24 hours, 48 hours, 72 hours, 96 hours and 120 hours post inoculation. At each time point, the optical density of the culture and secreted HSA were determined. The sample was then separated by centrifugation into a cell pellet and culture supernatant. The level of HSA secreted into the supernatant was measured by rocket gel electrophoresis and RNA extracted from the cell pellet. The RNA from each time point was separated into its individual components by gel electrophoresis; Northern blotted and probed with radiolabelled DNA homologous to the PGK and HSA structural genes. RNA was extracted from yeast cells as described by Linquist (Nature 293 (1981) 311-314). 10µg of total yeast RNA was resolved on a 1.0% agarose-formaldehyde gel and vacuum blotted from 20 x SSPE onto a Pall bio-dyne nylon membrane, and UV cross-linked according to Kroczek and Siebet (Anal. Biochem. 184 (1990) 90-95). Hybridisation was performed at 6 x SSPE, 5 x Denhardts, 0.1% (w/v) SDS, 100µg/ml denatured herring sperm DNA, at 50°C for 18 hours. Washing stringency was 0.2 x SSPE, 0.1 % (w/v) SDS, 50°C.

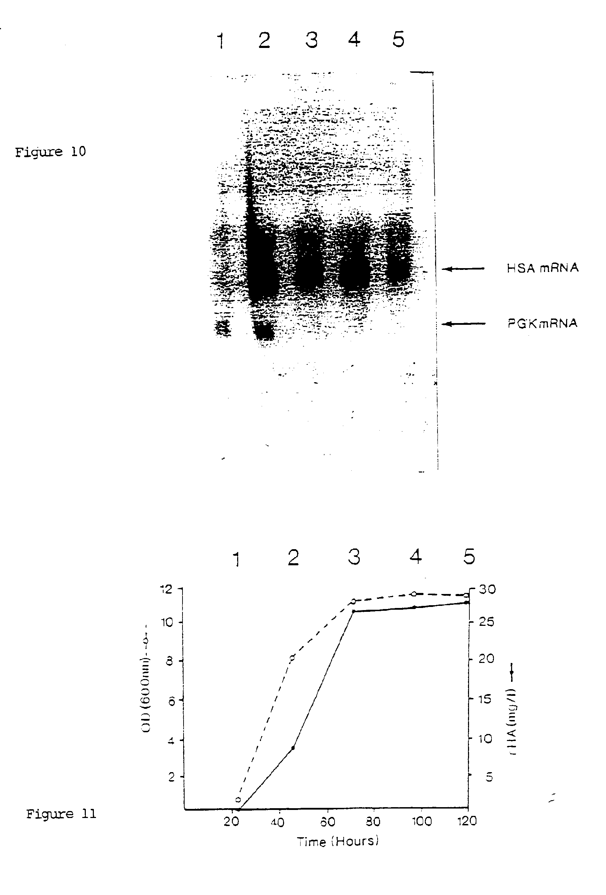

The results are illustrated in Figure 10. Figure 11 shows the optical density and level of rHA during the experiment. At the first timepoint 24 hrs post inoculation, PGK mRNA is observed; however, neither secreted HSA nor HSA mRNA are detected. At the second time point, 48 hrs post inoculation, both PGK and HSA mRNA are detected within the cell. The HSA mRNA is available for translation because secreted RSA is observed in the culture supernatant. At the next three time points, 72 hrs, 96 hrs and 120 hrs post inoculation, only HSA mRNA is observed and the PGK mRNA has disappeared. The level of HSA observed in the culture supernatant has increased from the previous time point, but no further increase is observed. The following conclusions can be drawn.
i) The HSA gene is not expressed during the early growth phase and does not mirror PGK expression.
ii) The HSA gene is expressed and HSA is secreted during the late logarithmic and stationary growth phase.
iii) HSA mRNA levels are maintained during stationary phase.

Furthermore, the timing of expression can be manipulated in the controlled environment of a fermentation vessel, be it batch, fed-batch or continuous culture. When repressing carbon sources such as sucrose or glucose are supplied as the sole carbon source, the expression of the heterologous protein is repressed. Consequently the growth of the host organism is not impaired by the synthesis of the heterologous protein. At a point predetermined by the operator the sucrose or glucose is replaced by a non-repressing carbon source such as glycerol or ethanol. Under these conditions the expression of the heterologous protein is de-repressed. Consequently production can be regulated in such a way as to optimise the synthesis of the desired product.

### EXAMPLE 3: Expression with various carbon sources

Strain 1 pAYE321 was grown for 72 hrs in 10 ml YEP, 200 rpm, 30°C supplemented with various carbon sources. In the control experiment sucrose is supplied instead of glucose but the final HSA secretion levels are identical. In all the other experiments a stimulation of HSA secretion is observed. The results are given in Table 1 below. The best carbon source would appear to be a combination of 1% (v/v) ethanol and 1% (v/v) glycerol. Although the stimulating effect at first sight does not appear very great it must be remembered that the value achieved on sucrose as a carbon source is really the value achieved on a mixture of sucrose and ethanol. If the culture is maintained in sucrose excess, the level of secreted HSA will be greatly reduced.

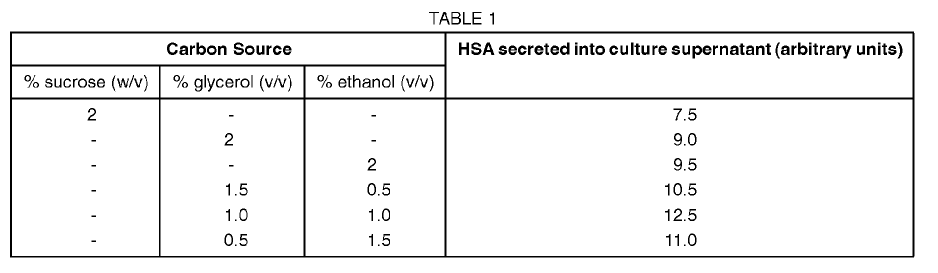

### EXAMPLE 4

This example describes plasmid pDVX4 designed for expressing and secreting the S. cerevisiae var diastaticus glucoamylase.

### Construction of Plasmid pDVX4

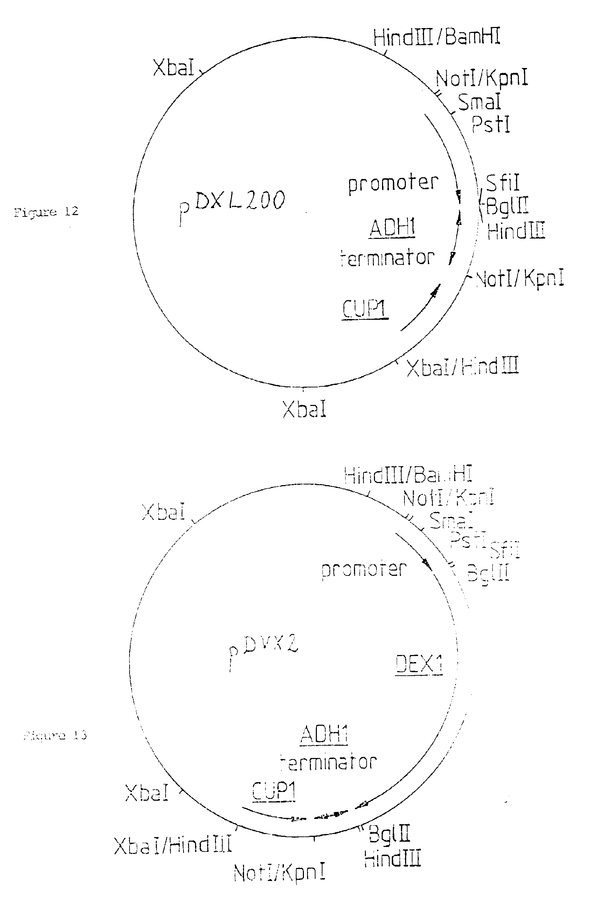

The initial step involved the construction of a generalised brewing yeast vector pDXL200 (Fig 12) which contained the following DNA sequences:
a) 0.34kbp Smal - SfiI fragment of the modified promoter (pAYE275).
b) A synthetic oligonucleotide linker containing restriction enzyme sites for SfiI, Bg/II and Hindlll:

### Synthetic Oligonucleotide Linker

The 5'-3' modified region and the two oligonucleotides are listed as SEQ14, SEQ8 and SEQ9 respectively
c) 0.45kbp of the ADH1 terminator (Hitzeman, R A et al. (1981). Nature. 293, 717.)
d) The CUP-1 gene and its flanking sequences from S. cerevisiae were present on 0.7kbp Kpnl - Xbal fragment and 0.38kbp BamHl - Kpnl fragment respectively (Karin, M et al, (1984). Proc. Natl. Acad. Sci. 81, 337.). The CUP-1 gene was used as a selective genetic marker for brewing yeast transformation.
e) 2.7kbp of bacterial DNA (pUC9) as present in pSAC3 (Chinery and Hinchliffe (1989) Curr. Genet. 16, 21-25).
f) 2µm DNA: 2. 2kbp HindIII fragment containing the 2µm origin of replication (Broach, J R (1982). The yeast plasmid 2µm circle. In "The Molecular Biology of the yeast Saccharomyces cerevisiae: Life Cycle and Inheritance". Eds. Strathern, J N, E W Jones and J R Broach). Cold Spring Harbor, p445.) The full DNA sequence of 2µm DNA is also known (Hartley, J L and Donelson, J E (1980). Nature. 226, 860).

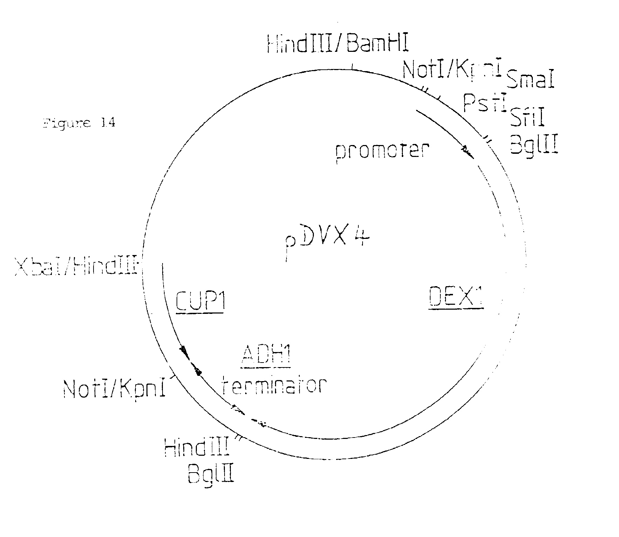

The DEX1 gene which codes for glucoamylase was isolated from S. cerevisiaevar diastaticus (Meaden P K et al, (1985) Gene. 34, 325 and PCT/GB85/00599; Pardo et al (1988 FEBS. Lett. 239, 179-184 describe the DEX1 promoter and part of the open reading frame). A 2.75kbp Bg/II fragment carrying the DEX1 gene was cloned into the unique Bg/II site in pDXL200 and the DNA sequence is represented as SEQ10, with the protein encoded thereby appearing as SEQ11. The resulting plasmid, pDVX2 (Fig. 13), was digested with Xbal to remove the smaller fragment (2.7kbp) containing the bacterial DNA. After gel purification, the larger Xbal fragment was transformed into brewing yeast and this plasmid designated pDVX4 (Fig. 14).

### Expression of DEX 1

Brewing yeast strains transformed to copper resistance with plasmid pDVX4 were assayed for glucoamylase production by measuring glucose released from starch using the hexokinase-UV assay (Boehringer-Mannheim). In all cases copper resistant transformants produced significant quantities of extracellular glucoamylase.
## Claims (Claims for the following Contracting State(s) : DE, FR, IT, NL, SE, CH, BE, AT, LU, GR, DK)

1. A promoter consisting of the wild-type Saccharomyces cerevisiae glycerol-3-phosphate dehydrogenase promoter having the nucleotide sequence defined herein as SEQ1 in isolation from the coding sequence which would normally neighbour the said promoter in wild-type Saccharomyces cerevisiae, or a variant or a functional portion of said promoter, wherein said variant or functional portion
(i) has at least 80% sequence homology with a region of SEQ1 which region is more homologous than any other region of SEQ1 to the variant or functional portion,
(ii) is at least 100 nucleotides long, and
(iii) either retains at least 80% of the ability of said wild-type promoter to promote transcription of a nucleotide sequence encoding a naturally occurring human serum albumin positioned downstream thereof or (a) retains at least 10% of the ability of said wild-type promoter to promote transcription of a nucleotide sequence encoding a naturally occurring human serum albumin positioned downstream thereof and (b) is repressed by complex carbon sources and derepressed by the absence of such sources.

2. A promoter according to Claim 1 being at least 200 nucleotides long.

3. A cloning vector or a yeast expression vector comprising a promoter according to any one of the preceding claims adjacent a restriction site such that a heterologous coding sequence may be located downstream of the promoter and in the correct reading frame in relation to a translational start codon.

4. A yeast expression vector according to Claim 3 comprising a heterologous coding sequence inserted as said.

5. A yeast expression vector according to Claim 4 wherein the heterologous coding sequence encodes human serum albumin or a variant or part thereof, optionally with a secretion leader sequence.

6. A yeast expression vector according to Claim 4 wherein the heterologous coding sequence encodes the glucoamylase of S. cerevisiae var diastaticus.

7. A yeast transformed with an expression vector according to Claim 4, 5 or 6.

8. A process for preparing a polypeptide, comprising fermenting a yeast according to Claim 7 and at least partially purifying the polypeptide expressed by the said heterologous coding sequence.

9. A process according to Claim 8 wherein the yeast is initially grown on a carbon source or sources which repress expression of the polypeptide and subsequently the carbon source is changed to a non-repressing compound or mixture of such compounds.

## Claims (Claims for the following Contracting State(s) : ES)

1. A process for preparing a promoter by conventional polynucleotide manipulation techniques, characterized in that the promoter consists of the wild-type Saccharomyces cerevisiae glycerol-3-phosphate dehydrogenase promoter having the nucleotide sequence defined herein as SEQ1 in isolation from the coding sequence which would normally neighbour the said promoter in wild-type Saccharomyces cerevisiae, or a variant or a functional portion of said promoter, wherein said variant or functional portion
(i) has at least 80% sequence homology with a region of SEQ1 which region is more homologous than any other region of SEQ1 to the variant or functional portion,
(ii) is at least 100 nucleotides long, and
(iii) either retains at least 80% of the ability of said wild-type promoter to promote transcription of a nucleotide sequence encoding a naturally occurring human serum albumin positioned downstream thereof or (a) retains at least 10% of the ability of said wild-type promoter to promote transcription of a nucleotide sequence encoding a naturally occurring human serum albumin positioned downstream thereof and (b) is repressed by complex carbon sources and derepressed by the absence of such sources.

2. A process according to Claim 1 wherein the promoter is at least 200 nucleotides long.

3. A process for preparing a cloning vector or a yeast expression vector comprising a promoter prepared according to any one of the preceding claims wherein the promoter is placed adjacent a restriction site such that a heterologous coding sequence may be located downstream of the promoter and in the correct reading frame in relation to a translational start codon.

4. A process according to Claim 3 additionally comprising inserting a heterologous coding sequence inserted as said.

5. A process according to Claim 4 wherein the heterologous coding sequence encodes human serum albumin or a variant or part thereof, optionally with a secretion leader sequence.

6. A process according to Claim 4 wherein the heterologous coding sequence encodes the glucoamylase of S. cerevisiae var diastaticus.

7. A process of transforming a yeast characterised in that the yeast is transformed with an expression vector according to Claim 4, 5 or 6.

8. A process for preparing a polypeptide, comprising fermenting a yeast prepared according to Claim 7 and at least partially purifying the polypeptide expressed by the said heterologous coding sequence.

9. A process according to Claim 8 wherein the yeast is initially grown on a carbon source or sources which repress expression of the polypeptide and subsequently the carbon source is changed to a non-repressing compound or mixture of such compounds.

## Patentansprüche (Patentansprüche für folgende(n) Vertragsstaat(en) : DE, FR, IT, NL, SE, CH, BE, AT, LU, GR, DK)

1. Promotor,bestehendausdemWildtypSaccharomycescerevisiaeGlycerin-3-phosphat-Dehydrogenase-Promotor mit der hierin als SEQ1 definierten Nucleotidsequenz isoliert von der Codiersequenz, die normalerweise dem Promotor im Wildtyp Saccharomyces cerevisiae benachbart wäre, oder einer Variante oder einem funktionellen Teil dieses Promotors, wobei die Variante oder der funktionelle Teil
(i) eine mindestens 80%ige Sequenzhomologie mit einem SEQ1-Bereich, der zu der Variante oder dem funktionellen Teil stärker homolog ist als irgendein anderer SEQ1-Bereich, aufweist;
(ii) mindestens 100 Nucleotide lang ist und
(iii) entweder mindestens 80% der Fähigkeit des Wildtyp-Promotors zur Promotion der Transkription einer Nucleotidsequenz mit Codierung für ein natürlich vorkommendes Humanserumalbumin in einer Position stromabwärts desselben behält oder (a) mindestens 10% der Fähigkeit des Wildtyp-Promotors zur Promotion der Transkription einer Nucleotidsequenz mit Codierung für ein natürlich vorkommendes Humanserumalbumin in einer Position stromabwärts desselben behält und (b) durch komplexe Kohlenstofflieferanten einer Repression unterliegt und bei Abwesenheit solcher Lieferanten von der Repression befreit ist.

2. Promotor nach Anspruch 1, der mindestens 200 Nucleotide lang ist.

3. Klonierungsvektor oder Hefeexpressionsvektor, umfassend einen Promotor nach einem der vorhergehenden Ansprüche in Nachbarschaft zu einer Restriktionsstelle dergestalt, daß stromabwärts vom Promotor und im korrekten Leserahmen in bezug auf ein Translationsstartcodon eine heterologe Codiersequenz angeordnet sein kann

4. Hefeexpressionsvektor nach Anspruch 3, umfassend eine - wie dargelegt - insertierte bzw eingefügte heterologe Codiersequenz

5. Hefeexpressionsvektor nach Anspruch 4, wobei die heterologe Codiersequenzfür Humanserumalbumin oder eine Variante oder einen Teil desselben, gegebenenfalls mit einer Sekretionsleadersequenz, kodiert.

6. Hefeexpressionsvektor nach Anspruch 4, wobei die heterologe Codiersequenz für die Glucoamylase von S. cerevisiae var. diastaticus codiert.

7. Hefe, welche mit einem Expressionsvektor nach Anspruch 4, 5 oder 6 transformiert ist

8. Verfahren zur Herstellung eines Polypeptids durch Fermentieren einer Hefe nach Anspruch 7 und zumindest Teilreinigen des durch die heterologe Codiersequenz exprimierten Polypeptids

9. Verfahren nach Anspruch 8, wobei die Hefe zunächst auf (einem) die Expression des Polypeptids unterdrückenden Kohlenstofflieferanten wachsengelassen wird und danach der Kohlenstofflieferant gegen eine nicht-unterdrückende Verbindung oder ein Gemisch solcher Verbindungen ausgetauscht wird.

## Patentansprüche (Patentansprüche für folgende(n) Vertragsstaat(en) : ES)

1. Verfahren zur Herstellung eines Promotors durch übliche Polynucleotidmanipulationsmaßnahmen, dadurch gekennzeichnet, daß der Promotor aus dem Wildtyp Saccharomyces cerevisiae Glycerin-3-phosphat-Dehydrogenase-Promotor mit der hierin als SEQ1 definierten Nucleotidsequenz isoliert von der Codiersequenz, die normalerweise dem Promotor im Wildtyp Saccharomyces cerevisiae benachbart wäre, oder einer Variante oder einem funktionellen Teil dieses Promotors, wobei die Variante oder der funktionelle Teil
(i) eine mindestens 80%ige Sequenzhomologie mit einem SEQ1-Bereich, der zu der Variante oder dem funktionellen Teil stärker homolog ist als irgendein anderer SEQ1-Bereich, aufweist;
(ii) mindestens 100 Nucleotide lang ist und
(iii) entweder mindestens 80% der Fähigkeit des Wildtyp-Promotors zur Promotion der Transkription einer Nucleotidsequenz mit Codierung für ein natürlich vorkommendes Humanserumalbumin in einer Position stromabwärts desselben behält oder (a) mindestens 10% der Fähigkeit des Wildtyp-Promotors zur Promotion der Transkription einer Nucleotidsequenz mit Codierung für ein natürlich vorkommendes Humanserumalbumin in einer Position stromabwärts desselben behält und (b) durch komplexe Kohlenstofflieferanten einer Repression unterliegt und bei Abwesenheit solcher Lierferanten von der Repression befreit ist,
besteht.

2. Verfahren nach Anspruch 1, wobei der Promotor mindestens 200 Nucleotide lang ist.

3. Verfahren zur Herstellung eines Klonierungsvektors oder eines Hefeexpressionsvektors, umfassend einen nach einem der vorhergehenden Ansprüche hergestellten Promotor, wobei der Promotor in Nachbarschaft zu einer Restriktionsstelle plaziert ist, dergestalt, daß stromabwärts vom Promotor und im korrekten Leserahmen in bezug auf ein Translationsstartcodon eine heterologe Codiersequenz angeordnet sein kann.

4. Verfahren nach Anspruch 3, bei welchem zusätzlich - wie dargelegt - eine heterologe Codiersequenz insertiert bzw. eingefügt wird.

5. Verfahren nach Anspruch 4, wobei die heterologe Codiersequenz für Humanserumalbumin oder eine Variante oder einen Teil desselben, gegebenenfalls mit einer Sekretionsleadersequenz, kodiert.

6. Verfahren nach Anspruch 4, wobei die heterologe Codiersequenz für die Glucoamylase von S. cerevisiae var. diastaticus codiert.

7. Verfahren zur Transformation einer Hefe, dadurch gekennzeichnet, daß die Hefe mit einem Expressionsvektor nach Anspruch 4, 5 oder 6 transformiert wird.

8. Verfahren zur Herstellung eines Polypeptids durch Fermentieren einer nach Anspruch 7 hergestellten Hefe und zumindestens Teilreinigen des durch die heterologe Codiersequenz exprimierten Polypeptids.

9. Verfahren nach Anspruch 8, wobei die Hefe zunächst auf (einem) die Expression des Polypeptids unterdrückenden Kohlenstofflieferanten wachsengelassen wird und danach der Kohlenstoff lieferant gegen eine nicht-unterdrükkende Verbindung oder ein Gemisch solcher Verbindungen ausgetauscht wird.

## Revendications (Revendications pour l'(les) Etat(s) contractant(s) suivant(s) : DE, FR, IT, NL, SE, CH, BE, AT, LU, GR, DK)

1. Promoteur consistant en le promoteur de la glycérol-3-phosphate déshydrogénase de Saccharomyces cerevisiae de type sauvage ayant la séquence de nucléotides définie ici en tant que SEQ1 isolée de la séquence codante qui est normalement voisine dudit promoteur dans Saccharomyces cerevisiae de type sauvage, ou une variante ou une partie fonctionnelle dudit promoteur, ladite variante ou partie fonctionnelle :
(i) ayant au moins 80 % d'homologie de séquence avec une région de SEQ1, laquelle région est plus homologue à la variante ou à la partie fonctionnelle qu'une quelconque autre région de SEQ1,
(ii) étant longue d'au moins 100 nucléotides, et
(iii) retenant au moins 80 % de la capacité dudit promoteur de type sauvage à favoriser la transcription d'une séquence de nucléotides codant pour une sérumalbumine humaine naturelle positionnée en aval de celui-ci ou (a) retenant au moins 10 % de la capacité dudit promoteur de type sauvage à favoriser la transcription d'une séquence de nucléotides codant pour une sérumalbumine humaine naturelle positionnée en aval de celui-ci et (b) étant réprimée par des sources de carbone complexes et déréprimée par l'absence de telles sources.

2. Promoteur selon la revendication 1, long d'au moins 200 nucléotides.

3. Vecteur de clonage ou vecteur d'expression pour levure, comprenant un promoteur selon l'une quelconque des revendications précédentes, adjacent à un site de restriction de sorte qu'une séquence codante hétérologue puisse être située en aval du promoteur et dans le cadre de lecture correct par rapport à un codon d'initiation de traduction.

4. Vecteur d'expression pour levure selon la revendication 3, comprenant une séquence codante hétérologue insérée comme décrit.

5. Vecteur d'expression pour levure selon la revendication 4, dans lequel la séquence codante hétérologue code pour la sérumalbumine humaine ou une variante ou partie de celle-ci, éventuellement avec une séquence leader de sécrétion.

6. Vecteur d'expression pour levure selon la revendication 4, dans lequel la séquence codante hétérologue code pour la glucoamylase de S. cerevisiae var diastaticus.

7. Levure transformée avec un vecteur d'expression selon la revendication 4, 5 ou 6.

8. Procédé pour préparer un polypeptide, comprenant la fermentation d'une levure selon la revendication 7 et la purification au moins partielle du polypeptide exprimé par ladite séquence codante hétérologue.

9. Procédé selon la revendication 8, dans lequel la levure est initialement développée sur une ou des source(s) de carbone qui réprime(nt) l'expression du polypeptide, après quoi la source de carbone est changée par un composé non répresseur ou un mélange de tels composés

## Revendications (Revendications pour l'(les) Etat(s) contractant(s) suivant(s) : ES)

1. Procédé de préparation d'un promoteur par des techniques de manipulation de polynucléotides conventionnelles, caractérisé en ce que le promoteur est constitué du promoteur glycérol-3-phosphate déshydrogénase de Saccharomyces cerevisiae de type sauvage ayant la séquence de nucléotides définie ici en tant que SEQ1 isolée de la séquence codante qui est normalement voisine dudit promoteur dans Saccharomyces cerevisiaede type sauvage, ou une variante ou une partie fonctionnelle dudit promoteur, ladite variante ou partie fonctionnelle :
(i) ayant au moins 80 % d'homologie de séquence avec une région de SEQ1, laquelle région est pius homologue à la variante ou à la partie fonctionnelle qu'une quelconque autre région de SEQ1,
(ii) étant longue d'au moins 100 nucléotides, et
(iii) retenant au moins 80 % de la capacité dudit promoteur de type sauvage à favoriser la transcription d'une séquence de nucléotides codant pour une sérumalbumine humaine naturelle positionnée en aval de celui-ci ou (a) retenant au moins 10 % de la capacité dudit promoteur de type sauvage à favoriser la transcription d'une séquence de nucléotides codant pour une sérumalbumine humaine naturelle positionnée en aval de celui-ci et (b) étant réprimée par des sources de carbone complexes et déréprimée par l'absence de telles sources.

2. Procédé selon la revendication 1, dans lequel le promoteur est long d'au moins 200 nucléotides.

3. Procédé pour préparer un vecteur de clonage ou un vecteur d'expression pour levure, comprenant un promoteur préparé selon l'une quelconque des revendications précédentes, dans lequel le promoteur est placé adjacent à un site de restriction de sorte qu'une séquence codante hétérologue puisse être située en aval du promoteur et dans le cadre de lecture correct par rapport à un codon d'initiation de traduction.

4. Procédé selon la revendication 3, comprenant en plus l'insertion d'une séquence codante hétérologue insérée comme décrit.

5. Procédé selon la revendication 4, dans lequel la séquence codante hétérologue code pour la sérumalbumine humaine ou une variante ou partie de celle-ci, éventuellement avec une séquence leader de sécrétion.

6. Procédé selon la revendication 4, dans lequel la séquence codante hétérologue code pour la glucoamylase de S. cerevisiae var diastaticus.

7. Procédé pour transformer une levure, caractérisé en ce que la levure est transformée avec un vecteur d'expression selon la revendication 4, 5 ou 6.

8. Procédé pour préparer un polypeptide, comprenant la fermentation d'une levure selon la revendication 7 et la purification au moins partielle du polypeptide exprimé par ladite séquence codante hétérologue.

9. Procédé selon la revendication 8, dans lequel la levure est initialement développée sur une ou des source(s) de carbone qui réprime(nt) l'expression du polypeptide, après quoi la source de carbone est changée par un composé non répresseur ou un mélange de tels composés.


(2) INFORMATION FOR SEQ ID NO:1:
   (i) SEQUENCE CHARACTERISTICS :
      (A) LENGTH: 1357 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: N
   (iv) ANTI-SENSE: N
   (vi) ORIGINAL SOURCE:
      (A) ORGANISM: Saccharomyces cerevisiae
   (ix) FEATURE:
      (A) NAME/KEY: promoter
      (B) LOCATION: 42..52
      (D) OTHER INFORMATION: /function= "RNA POLIII promoter box A"
   (ix) FEATURE:
      (A) NAME/KEY: promoter
      (B) LOCATION: 86.96
      (D) OTHER INFORMATION: /function= "RNA POLIII Promoter box B"
   (ix) FEATURE
      (A) NAME/KEY: promoter
      (B) LOCATION: 113..118
      (D) OTHER INFORMATION: /function= "RNA POLIII Terminator"
   (ix) FEATURE:
      (A) NAME/KEY: protein_bind
      (B) LOCATION: 1091..1103
      (D) OTHER INFORMATION: /bound_moiety= "RAPI/GRFI/TUFI"
   (ix) FEATURE:
      (A) NAME/KEY: protein_bind
      (B) LOCATION: 1106..1118
      (D) OTHER INFORMATION: /bound_moiety= "RAPI/GRFI/TUFI"
   (ix) FEATURE:
      (A) NAME/KEY: misc_signal
      (B) LOCATION: 1176..1241
      (D) OTHER INFORMATION: /function= "Pyrimidine (CT) block"
   (ix) FEATURE:
      (A) NAME/KEY: TATA_signal
      (B) LOCATION: 1326..1335
      (D) OTHER INFORMATION:
   (ix) FEATURE
      (A) NAME/KEY: misc_signal
      (B) LOCATION: 1369..1406
      (D) OTHER INFORMATION: /function= "Pyrimidine (CT) block"
   (ix) FEATURE:
      (A) NAME/KEY: misc_signal
      (B) LOCATION: 1418..1421
      (D) OTHER INFORMATION: /function= "CRAG box"
   (ix) FEATURE:
      (A) NAME/KEY: misc_signal
      (B) LOCATION: 1425..1429
      (D) OTHER INFORMATION: /function= "CCAAT box"
   (ix) FEATURE:
      (A) NAME/KEY: misc_feature
      (B) LOCATION: 1031..1036
      (D) OTHER INFORMATION: /function= "Pstl retriction site"
   (ix) FEATURE:
      (A) NAME/KEY: misc_feature
      (B) LOCATION: 1314..1317
      (D) OTHER INFORMATION:
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 1483 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: misc feature
      (B) LOCATION: 123..124
      (D) OTHER INFORMATION: /function= "Sstll restriction site"
   (xi) SEQUENCE DESCRIPTION: SEQ ID NO:2:
   AAGAAAGATT CTCGGTAACG ACCATACAAA TATTGGGCGT GTGGCGTAGT CGGTAGCGCG 60
   CTCCCTTAGC ATGGGAGAGG TCTCCGGTTC GATTCCGGAC TCGTCCAAAT TATTTTTTAC 120
   TTTCCGCGGT GCCGAGATGC AGACGTGGCC AACTGTGTCT GCCGTCGCAA AATGATTTGA    180
   ATTTTGCGTC GCGCACGTTT CTCACGTACA TAATAAGTAT TTTCATACAG TTCTAGCAAG    240
   ACGAGGTGGT CAAAATAGAA GCGTCCTATG TTTTACAGTA CAAGACAGTC CATACTGAAA    300
   TGACAACGTA CTTGACTTTT CAGTATTTTC TTTTTCTCAC AGTCTGGTTA TTTTTGAAAG    360
   CGCACGAAAT ATATGTAGGC AAGCATTTTC TGAGTCTGCT GACCTCTAAA ATTAATGCTA    420
   TTGTGCACCT TAGTAACCCA AGGCAGGACA GTTACCTTGC GTGGTGTTAC TATGGCCGGA    480
   AGCCCGAAAG AGTTATCGTT ACTCCGATTA TTTTGTACAG CTGATGGGAC CTTGCCGTCT    540
   TCATTTTTTT TTTTTTTCAC CTATAGAGCC GGGCAGAGCT GCCCGGCTTA ACTAAGGGCC    600
   GGAAAAAAAA CGGAAAAAAG AAAGCCAAGC GTGTAGACGT AGTATAACAG TATATCTGAC    660
   ACCCACGTGA TGACCACGTA ATCGCATCGC CCCTCACCTC TCACCTCTCA CCGCTGACTC    720
   AGCTTCACTA AAAAGGAAAA TATATACTCT TTCCCAGGCA AGGTGACAGC GGTCCCCGTC    780
   TCCTCCACAA AGGCCTCTCC TGGGGTTTGA GCAAGTCTAA GTTTACGTAG CATAAAAATT    840
   CTCGGATTGC GTCAAATAAT AAAAAAAGTA ACCCCACTTC TACTTCTACA TCGGAAAAAC    900
   ATTCCATTCA CATATCGTCT TTGGCCTATC TTGTTTTGTC CTCGGTAGAT CAGGTCAGTA    960
   CAAACGCAAC ACGAAAGAAC AAAAAAAGAA GAAAACAGAA GGCCAAGACA GGGTCAATGA   1020
   GACTGTTGTC CTCCTACTGT CCCTATGTCT CTGGCCGATC ACGCGCCATT GTCCCTCAGA   1080
   AACAAATCAA ACACCCACAC CCCGGGCACC CAAAGTCCCC ACCCACACCA CCAATACGTA   1140
   AACGGGGCGC CCCCTGCAGG CCCTCCTGCG CGCGGCCTCC CGCCTTGCTT CTCTCCCCTT   1200
   CCTTTTCTTT TTCCAGTTTT CCCTATTTTG TCCCTTTTTC CGCACAACAA GTATCAGAAT   1260
   GGGTTCATCA AATCTATCCA ACCTAATTCG CACGTAGACT GGCTTGGTAT TGGCAGTTTC   1320
   GTAGTTATAT ATATACTACC ATGAGTGAAA CTGTTACGTT ACCTTAAATT CTTTCTCCCT   1380
   TTAATTTTCT TTTATCTTAC TCTCCTACAT AAGACATCAA GAAACAATTG TATATTGTAC   1440
   ACCCCCCCCC TCCACAAACA CAAATATTGA TAATATAAAG ATG                      1483
(2) INFORMATION FOR SEQ ID NO:3:
   (i) SEQUENCE CHARACTERISTICS:
      (A) LENGTH: 380 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: N
   (iv) ANTI-SENSE N
   (ix) FEATURE:
      (A) NAME/KEY: misc_feature
      (B) LOCATION: 54..59
      (D) OTHER INFORMATION: /function= "Pstl restriction site"
   (ix) FEATURE
      (A) NAME/KEY: misc_feature
      (B) LOCATION: 54..59
      (D) OTHER INFORMATION: /function= "Pstl restriction site"
   (ix) FEATURE:
      (A) NAME/KEY: misc_feature
      (B) LOCATION: 337..340
      (D) OTHER INFORMATION: /function= "Rsal restriction site"
   (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 24 amino acids
      (B) TYPE: amino acid
      (C) STRANDEDNESS: single
      (D) TOPOLOGY: linear
   (ii) MOLECULE TYPE: peptide
   (iii) HYPOTHETICAL: N
   (iv) ANTI-SENSE: N
   (v) FRAGMENT TYPE: N-terminal
   (ix) FEATURE:
      (A) NAME/KEY: Peptide
      (B) LOCATION: 1..24
      (D) OTHER INFORMATION: /label= leader /note= "Synthetic secretion leader sequence"
   (xi) SEQUENCE DESCRIPTION: SEQ ID NO:4:
   Met Lys Trp Val Ser Phe Ile Ser Leu Leu Phe Leu Phe Ser Ser Ala
   1               5                   10                  15
   Tyr Ser Arg Ser Leu Asp Lys Arg
               20
(2) INFORMATION FOR SEQ ID NO:5:
   (i) SEQUENCE CHARACTERISTICS:
      (A) LENGTH: 21 amino acids
      (B) TYPE: amino acid
      (C) STRANDEDNESS: single
      (D) TOPOLOGY: linear
   (ii) MOLECULE TYPE: peptide
   (iii) HYPOTHETICAL: N
   (iv) ANTI-SENSE: N
   (v) FRAGMENT TYPE: N-terminal
   (ix) FEATURE:
      (A) NAME/KEY: Peptide
      (B) LOCATION: 1..21
      (D) OTHER INFORMATION: /label= leader /note= "Synthetic secretion leader sequence"
   (xi) SEQUENCE DESCRIPTION: SEQ ID NO:5:
   Met Asn Ile Phe Tyr Ile Phe Leu Phe Leu Leu Ser Phe Val Gln Gly
   1               5                   10                  15
   Ser Leu Asp Lys Arg
               20
(2) INFORMATION FOR SEQ ID NO:6:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: N
   (iv) ANTI-SENSE: N
   (vi) ORIGINAL SOURCE:
      (A) ORGANISM: Saccharomyces cerevisiae
   (ix) FEATURE:
      (A) NAME/KEY: exon
      (B) LOCATION: 1..47
      (D) OTHER INFORMATION: /note= "Natural ATG environment of the promoter"
   (xi) SEQUENCE DESCRIPTION: SEQ ID NO:6:
   GTACACCCCC CCCCTCCACA AACACAAATA TTGATAATAT AAAGATG 47
(2) INFORMATION FOR SEQ ID NO:7:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: N
   (iv) ANTI-SENSE: N
   (ix) FEATURE
      (A) NAME/KEY: modified_base
      (B) LOCATION: 5
      (D) OTHER INFORMATION:
   (ix) FEATURE
      (A) NAME/KEY: modified_base
      (B) LOCATION: 6
      (D) OTHER INFORMATION:
   (ix) FEATURE:
      (A) NAME/KEY: modified_base
      (B) LOCATION: 14
      (D) OTHER INFORMATION:
   (ix) FEATURE:
      (A) NAME/KEY: modified_base
      (B) LOCATION: 15
      (D) OTHER INFORMATION:
   (ix) FEATURE:
      (A) NAME/KEY: -
      (B) LOCATION: 5..17
      (D) OTHER INFORMATION: /label= Sfil /note= "Sfil restriction site"
   (xi) SEQUENCE DESCRIPTION: SEQ ID NO:7
(2) INFORMATION FOR SEQ ID NO:8:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: Y
   (iv) ANTI-SENSE: N
   (ix) FEATURE
      (A) NAME/KEY: misc_feature
      (B) LOCATION: 1..12
      (D) OTHER INFORMATION: /function= "synthetic oligo used to create SEQ14"
   (xi) SEQUENCE DESCRIPTION: SEQ ID NO:8:
   CGGCCAGATC TA 12
(2) INFORMATION FOR SEQ ID NO:9:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: misc feature
      (B) LOCATION: 1..19
      (D) OTHER INFORMATION: /function= "synthetic oligo used to create SEQ14" /note= "This oligo is complementary to SEQB"
   (xi) SEQUENCE DESCRIPTION: SEQ ID NO:9:
   AGCTTAGATC TGGCCGGGG 19
(2) INFORMATION FOR SEQ ID NO:10:
   (i) SEQUENCE CHARACTERISTICS:
      (A) LENGTH: 2754 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: N
   (iv) ANTI-SENSE N
   (vi) ORIGINAL SOURCE:
      (A) ORGANISM: Saccharomyces cerevisiae
      (B) STRAIN: S. cerevisiae var. diastaticus 5106-9A
   (ix) FEATURE:
      (A) NAME/KEY: misc_feature
      (B) LOCATION: 98..103
      (D) OTHER INFORMATION: /function= "Stull/Bglll site"
   (ix) FEATURE:
      (A) NAME/KEY: CDS
      (B) LOCATION: 126..2543
      (D) OTHER INFORMATION:
   (xi) SEQUENCE DESCRIPTION: SEQ ID NO:10:
   GATCTTTTGC TTCCTAAACT AAACCTATAA AAAGCACCCT ATTCATCAGT TATAATCTCT      60
   TGTCATGTTG TGGTTCTAAT TGAAAATATA CTATGGTAGG CCTCAAAAAT CCATATACGC     120
   ACACT ATG CAA AGA CCA TTT CTA CTC GCT TAT TTG GTC CTT TCG CTT       167
         Met Gln Arg Pro Phe Leu Leu Ala Tyr Leu Val Leu Ser Leu
          1               5                   10
   CTA TTT AAC TCA GCT TTG GGT TTT CCA ACT GCA CTA GTT CCT AGA GGA     215
   Leu Phe Asn Ser Ala Leu Gly Phe Pro Thr Ala Leu Val Pro Arg Gly
   15                  20                  25                  30
   TCC TCC TCT AGC AAC ATC ACT TCG TCC GGT CCA TCT TCA ACT CCA TTC     263
   Ser Ser Ser Ser Asn Ile Thr Ser Ser Gly Pro Ser Ser Thr Pro Phe
                   35                  40                  45
   AGC TCT GCT ACT GAA AGC TTT TCT ACT GGC ACT ACT GTC ACT CCA TCA     311
   Ser Ser Ala Thr Glu Ser Phe Ser Thr Gly Thr Thr Val Thr Pro Ser
               50                  55                  60
   TCA TCC AAA TAC CCT GGC AGT AAA ACA GAA ACT TCT GTT TCT TCT ACA     359
   Ser Ser Lys Tyr Pro Gly Ser Lys Thr Glu Thr Ser Val Ser Ser Thr
           65                  70                  75
   ACC GAA ACT ACC ATT GTT CCA ACT ACA ACT ACG ACT TCT GTC ATA ACA     407
   Thr Glu Thr Thr Ile Val Pro Thr Thr Thr Thr Thr Ser Val Ile Thr
       80                  85                  90
   CCA TCA ACA ACC ACT ATT ACC ACT ACG GTT TGC TCT ACA GGA ACA AAC     455
   Pro Ser Thr Thr Thr Ile Thr Thr Thr Val Cys Ser Thr Gly Thr Asn
   95                  100                 105                 110
   TCT GCC GGT GAA ACT ACT TCT GGA TGC TCT CCA AAG ACC ATT ACA ACT     503
   Ser Ala Gly Glu Thr Thr Ser Gly Cys Ser Pro Lys Thr Ile Thr Thr
                   115                 120                 125
   ACT GTT CCA TGT TCA ACC AGT CCA AGC GAA ACC GCA TCG GAA TCA ACA     551
   Thr Val Pro Cys Ser Thr Ser Pro Ser Glu Thr Ala Ser Glu Ser Thr
               130                 135                 140
   ACC ACT TCA CCT ACC ACA CCT GTA ACT ACA GTT GTC GCA ACC ACC GTC     599
   Thr Thr Ser Pro Thr Thr Pro Val Thr Thr Val Val Ala Thr Thr Val
           145                 150                 155
   GTT ACT ACT GAG TAT TCT ACT AGT ACA AAA CAA GGT GGT GAA ATT ACA     647
   Val Thr Thr Glu Tyr Ser Thr Ser Thr Lys Gln Gly Gly Glu Ile Thr
       160                 165                 170
   ACT ACA TTT GTC ACC AAA AAC AGT CCA ACC ACT TAC CTA ACT ACA ATT     695
   Thr Thr Phe Val Thr Lys Asn Ser Pro Thr Thr Tyr Leu Thr Thr Ile
   175                 180                 185                 190
   GCT CCA ACT TCA TCA GTC ACT ACG GTT ACC AAT TTC ACC CCA ACC ACT     743
   Ala Pro Thr Ser Ser Val Thr Thr Val Thr Asn Phe Thr Pro Thr Thr
                   195                 200                 205
   ATT ACT ACT ACG GTT TGC TCT ACA GGA ACA AAC TCT GCC GGT GAA ACT     791
   Ile Thr Thr Thr Val Cys Ser Thr Gly Thr Asn Ser Ala Gly Glu Thr
               210                 215                 220
   ACC TCT GGA TGC TCT CCA AAG ACT GTC ACA ACA ACT GTT CTT TGT TCA     839
   Thr Ser Gly Cys Ser Pro Lys Thr Val Thr Thr Thr Val Leu Cys Ser
           225                 230                 235
   ACT GGT ACT GGC GAA TAC ACT ACT GAA GCT ACC GCC CCT GTT ACA ACA        887
   Thr Gly Thr Gly Glu Tyr Thr Thr Glu Ala Thr Ala Pro Val Thr Thr
       240                 245                 250
   GCT GTC ACA ACC ACC GTT GTT ACC ACT GAA TCC TCT ACG GGT ACT AAC        935
   Ala Val Thr Thr Thr Val Val Thr Thr Glu Ser Ser Thr Gly Thr Asn
   255                 260                 265                 270
   TCC GTC GGT AAG ACG ACA ACT AGT TAC ACA ACA AAG TCT GTA CCA ACC        983
   Ser Val Gly Lys Thr Thr Thr Ser Tyr Thr Thr Lys Ser Val Pro Thr
                   275                 280                 285
   ACC TAT GTA TTT GAC TTT GGC AAG GGC ATT CTC GAT CAA AGC TGC GGC       1031
   Thr Tyr Val Phe Asp Phe Gly Lys Gly Ile Leu Asp Gln Ser Cys Gly
               290                 295                 300
   GGT GTA TTT TCA AAC AAC GGC TCT TCG CAA GTG CAG CTG CGG GAT GTA       1079
   Gly Val Phe Ser Asn Asn Gly Ser Ser Gln Val Gln Leu Arg Asp Val
           305                 310                 315
   GTC TTG ATG AAT GGG ACA GTG GTA TAC GAT TCA AAC GGC GCT TGG GAC       1127
   Val Leu Met Asn Gly Thr Val Val Tyr Asp Ser Asn Gly Ala Trp Asp
       320                 325                 330
   AGT AGT GCG CTG GAG GAG TGG CTC CAG CGA CAG AAA AAA GTT TCC ATC       1175
   Ser Ser Ala Leu Glu Glu Trp Leu Gln Arg Gln Lys Lys Val Ser Ile
   335                 340                 345                 350
   GAA AGA ATA TTT GAA AAT ATT GGG CCC AGC GCC GTG TAT CCG TCT ATT       1223
   Glu Arg Ile Phe Glu Asn Ile Gly Pro Ser Ala Val Tyr Pro Ser Ile
                   355                 360                 365
   TTG CCT GGG GTC GTG ATT GCG TCA CCA TCG CAA ACG CAT CCA GAC TAC       1271
   Leu Pro Gly Val Val Ile Ala Ser Pro Ser Gln Thr His Pro Asp Tyr
               370                 375                 380
   TTC TAC CAA TGG ATA AGG GAC AGC GCG TTG ACG ATA AAC AGT ATT GTC       1319
   Phe Tyr Gln Trp Ile Arg Asp Ser Ala Leu Thr Ile Asn Ser Ile Val
           385                 390                 395
   TCT CAT TCT GCG GAC CCG GCA ATA GAG ACG TTA TTG CAG TAC CTG AAC       1367
   Ser His Ser Ala Asp Pro Ala Ile Glu Thr Leu Leu Gln Tyr Leu Asn
       400                 405                 410
   GTT TCA TTC CAC TTG CAA AGA ACC AAC AAC ACA TTG GGC GCT GGC ATT       1415
   Val Ser Phe His Leu Gln Arg Thr Asn Asn Thr Leu Gly Ala Gly Ile
   415                 420                 425                 430
   GGT TAC ACT AAC GAT ACA GTG GCT TTG GGA GAC CCT AAG TGG AAC GTC       1463
   Gly Tyr Thr Asn Asp Thr Val Ala Leu Gly Asp Pro Lys Trp Asn Val
                   435                 440                 445
   GAC AAC ACG GCT TTC ACG GAA CCT TGG GGT CGT CCT CAA AAC GAT GGC       1511
   Asp Asn Thr Ala Phe Thr Glu Pro Trp Gly Arg Pro Gln Asn Asp Gly
               450                 455                 460
   CCT GCT CTT CGA AGC ATT GCC ATC TTA AAA ATC ATC GAC TAC ATC AAG       1559
   Pro Ala Leu Arg Ser Ile Ala Ile Leu Lys Ile Ile Asp Tyr Ile Lys
           465                 470                 475
   CAA TCT GGC ACT GAT CTG GGG GCC AAG TAC CCA TTC CAG TCC ACC GCA 1607
   Gln Ser Gly Thr Asp Leu Gly Ala Lys Tyr Pro Phe Gln Ser Thr Ala
   480 485 490
   GAT ATC TTT GAT GAT ATT GTA CGT TGG TAC CTG AGG TTC ATT ATT GAC 1655
   Asp Ile Phe Asp Asp Ile Val Arg Trp Tyr Leu Arg Phe Ile Ile Asp
   495 500 505 510
   CAC TGG AAT TCT TCC GGA TTT GAT CTA TGG GAG GAA GTC AAT GGC ATG 1703
   His Trp Asn Ser Ser Gly Phe Asp Leu Trp Glu Glu Val Asn Gly Met
   515 520 525
   CAT TTC TTT ACT TTA CTG GTA CAA CTG TCT GCA GTG GAC AGG ACG CTG 1751
   His Phe Phe Thr Leu Leu Val Gln Leu Ser Ala Val Asp Arg Thr Leu
   530 535 540
   TCG TAT TTT AAC GCC TCA GAA CGG TCG TCT CCC TTT GTT GAA GAA TTG 1799
   Ser Tyr Phe Asn Ala Ser Glu Arg Ser Ser Pro Phe Val Glu Glu Leu
   545 550 555
   CGT CAG ACA CGC CGG GAC ATC TCC AAG TTT TTA GTG GAC CCT GCG AAT 1847
   Arg Gln Thr Arg Arg Asp Ile Ser Lys Phe Leu Val Asp Pro Ala Asn
   560 565 570
   GGG TTT ATC AAC GGC AAG TAC AAT TAT ATT GTT GAG ACA CCC ATG ATT 1895
   Gly Phe Ile Asn Gly Lys Tyr Asn Tyr Ile Val Glu Thr Pro Met Ile
   575 580 585 590
   GCC GAC ACA TTG AGA TCC GGA CTG GAC ATA TCC ACT TTA TTA GCT GCG 1943
   Ala Asp Thr Leu Arg Ser Gly Leu Asp Ile Ser Thr Leu Leu Ala Ala
   595 600 605
   AAC ACC GTC CAC GAT GCG CCA TCT GCT TCC CAT CTT CCG TTC GAT ATC 1991
   Asn Thr Val His Asp Ala Pro Ser Ala Ser His Leu Pro Phe Asp Ile
   610 615 620
   AAT GAC CCT GCC GTC CTG AAC ACG TTG CAC CAT TTG ATG TTG CAC ATG 2039
   Asn Asp Pro Ala Val Leu Asn Thr Leu His His Leu Met Leu His Met
   625 630 635
   CGT TCG ATA TAC CCC ATC AAC GAT AGC TCC AAA AAT GCA ACG GGT ATT 2087
   Arg Ser Ile Tyr Pro Ile Asn Asp Ser Ser Lys Asn Ala Thr Gly Ile
   640 645 650
   GCC CTG GGG CGG TAT CCT GAG GAC GTA TAT GAT GGA TAT GGC GTT GGC 2135
   Ala Leu Gly Arg Tyr Pro Glu Asp Val Tyr Asp Gly Tyr Gly Val Gly
   655 660 665 670
   GAG GGA AAT CCC TGG GTC CTG GCC ACG TGT GCC GCT TCA ACA ACG CTT 2183
   Glu Gly Asn Pro Trp Val Leu Ala Thr Cys Ala Ala Ser Thr Thr Leu
   675 680 685
   TAT CAG CTC ATT TAC AGA CAC ATC TCT GAG CAG CAT GAC TTG GTT GTC 2231
   Tyr Gln Leu Ile Tyr Arg His Ile Ser Glu Gln His Asp Leu Val Val
   690 695 700
   CCA ATG AAC AAC GAT TGT TCG AAC GCA TTT TGG AGC GAG CTG GTA TTC 2279
   Pro Met Asn Asn Asp Cys Ser Asn Ala Phe Trp Ser Glu Leu Val Phe
   705 710 715
   TCC AAC CTC ACG ACT TTG GGA AAT GAC GAA GGC TAT TTG ATT TTG GAG      2327
   Ser Asn Leu Thr Thr Leu Gly Asn Asp Glu Gly Tyr Leu Ile Leu Glu
       720                 725                 730
   TTC AAT ACA CCT GCC TTC AAT CAA ACC ATA CAA AAA ATC TTC CAA CTA      2375
   Phe Asn Thr Pro Ala Phe Asn Gln Thr Ile Gln Lys Ile Phe Gln Leu
   735                 740                 745                 750
   GCT GAT TCA TTC TTG GTC AAG CTG AAA GCC CAC GTG GGA ACA GAC GGG      2423
   Ala Asp Ser Phe Leu Val Lys Leu Lys Ala His Val Gly Thr Asp Gly
                   755                 760                 765
   GAA CTA AGT GAA CAA TTT AAC AAA TAC ACA GGG TTT ATG CAG GGT GCC      2471
   Glu Leu Ser Glu Gln Phe Asn Lys Tyr Thr Gly Phe Met Gln Gly Ala
               770                 775                 780
   CAA CAC CTT ACC TGG TCC TAT ACT TCA TTC TGG GAT GCC TAT CAA ATA      2519
   Gln His Leu Thr Trp Ser Tyr Thr Ser Phe Trp Asp Ala Tyr Gln Ile
           785                 790                 795
   AGA CAA GAA GTT TTA CAG AGT TTG TAGACAAAAA AAAATAAAAG AAAAGCGAGA     2573
   Arg Gln Glu Val Leu Gln Ser Leu
       800                 805
   AGTATACACA AGTGTATTTC CTAGATATTT ACATCAAATA TATATATATA TACTTATTTA    2633
   CAAAACTCTG ATATTATAAA TTAATTAGAT AACTATGTCG GAACGTCCAG CCCAACCACG    2693
   TTTGCAGTTC TTTTCACTTT CTCATCCTGT GTCAACTTGT TGCCGGATTG TATCTGTCGA    2753
   C                                                                    2754
(2) INFORMATION FOR SEQ ID NO:11:
   (i) SEQUENCE CHARACTERISTICS:
      (A) LENGTH: 806 amino acids
      (B) TYPE: amino acid
      (D) TOPOLOGY: linear
   (ii) MOLECULE TYPE: protein
   (xi) SEQUENCE DESCRIPTION: SEQ ID NO:11:
   Met Gln Arg Pro Phe Leu Leu Ala Tyr Leu Val Leu Ser Leu Leu Phe
     1               5                  10                  15
   Asn Ser Ala Leu Gly Phe Pro Thr Ala Leu Val Pro Arg Gly Ser Ser
               20                  25                  30
   Ser Ser Asn Ile Thr Ser Ser Gly Pro Ser Ser Thr Pro Phe Ser Ser
           35                  40                  45
   Ala Thr Glu Ser Phe Ser Thr Gly Thr Thr Val Thr Pro Ser Ser Ser
       50                  55                  60
   Lys Tyr Pro Gly Ser Lys Thr Glu Thr Ser Val Ser Ser Thr Thr Glu
   65                  70                  75                  80
   Thr Thr Ile Val Pro Thr Thr Thr Thr Thr Ser Val Ile Thr Pro Ser
                       85                                      90                                      95
   Thr Thr Thr Ile Thr Thr Thr Val Cys Ser Thr Gly Thr Asn Ser Ala
               100                 105                 110
   Gly Glu Thr Thr Ser Gly Cys Ser Pro Lys Thr Ile Thr Thr Thr Val
           115                 120                 125
   Pro Cys Ser Thr Ser Pro Ser Glu Thr Ala Ser Glu Ser Thr Thr Thr
       130                 135                 140
   Ser Pro Thr Thr Pro Val Thr Thr Val Val Ala Thr Thr Val Val Thr
   145                 150                 155                 160
   Thr Glu Tyr Ser Thr Ser Thr Lys Gln Gly Gly Glu Ile Thr Thr Thr
                   165                 170                 175
   Phe Val Thr Lys Asn Ser Pro Thr Thr Tyr Leu Thr Thr Ile Ala Pro
               180                 185                 190
   Thr Ser Ser Val Thr Thr Val Thr Asn Phe Thr Pro Thr Thr Ile Thr
           195                 200                 205
   Thr Thr Val Cys Ser Thr Gly Thr Asn Ser Ala Gly Glu Thr Thr Ser
       210                 215                 220
   Gly Cys Ser Pro Lys Thr Val Thr Thr Thr Val Leu Cys Ser Thr Gly
   225                 230                 235                 240
   Thr Gly Glu Tyr Thr Thr Glu Ala Thr Ala Pro Val Thr Thr Ala Val
                   245                 250                 255
   Thr Thr Thr Val Val Thr Thr Glu Ser Ser Thr Gly Thr Asn Ser Val
               260                 265                 270
   Gly Lys Thr Thr Thr Ser Tyr Thr Thr Lys Ser Val Pro Thr Thr Tyr
           275                 280                 285
   Val Phe Asp Phe Gly Lys Gly Ile Leu Asp Gln Ser Cys Gly Gly Val
       290                 295                 300
   Phe Ser Asn Asn Gly Ser Ser Gln Val Gln Leu Arg Asp Val Val Leu
   305                 310                 315                 320
   Met Asn Gly Thr Val Val Tyr Asp Ser Asn Gly Ala Trp Asp Ser Ser
                   325                 330                 335
   Ala Leu Glu Glu Trp Leu Gln Arg Gln Lys Lys Val Ser Ile Glu Arg
               340                 345                 350
   Ile Phe Glu Asn Ile Gly Pro Ser Ala Val Tyr Pro Ser Ile Leu Pro
           355                 360                 365
   Gly Val Val Ile Ala Ser Pro Ser Gln Thr His Pro Asp Tyr Phe Tyr
       370                 375                 380
   Gln Trp Ile Arg Asp Ser Ala Leu Thr Ile Asn Ser Ile Val Ser His
   385                 390                 395                 400
   Ser Ala Asp Pro Ala Ile Glu Thr Leu Leu Gln Tyr Leu Asn Val Ser
                       405                                     410                                     415
   Phe His Leu Gln Arg Thr Asn Asn Thr Leu Gly Ala Gly Ile Gly Tyr
                   420                     425                     430
   Thr Asn Asp Thr Val Ala Leu Gly Asp Pro Lys Trp Asn Val Asp Asn
               435                     440                     445
   Thr Ala Phe Thr Glu Pro Trp Gly Arg Pro Gln Asn Asp Gly Pro Ala
       450                     455                     460
   Leu Arg Ser Ile Ala Ile Leu Lys Ile Ile Asp Tyr Ile Lys Gln Ser
   465                     470                     475                     480
   Gly Thr Asp Leu Gly Ala Lys Tyr Pro Phe Gln Ser Thr Ala Asp Ile
                   485                     490                     495
   Phe Asp Asp Ile Val Arg Trp Tyr Leu Arg Phe Ile Ile Asp His Trp
               500                     505                     510
   Asn Ser Ser Gly Phe Asp Leu Trp Glu Glu Val Asn Gly Met His Phe
           515                     520                     525
   Phe Thr Leu Leu Val Gln Leu Ser Ala Val Asp Arg Thr Leu Ser Tyr
       530                     535                     540
   Phe Asn Ala Ser Glu Arg Ser Ser Pro Phe Val Glu Glu Leu Arg Gln
   545                     550                     555                     560
   Thr Arg Arg Asp Ile Ser Lys Phe Leu Val Asp Pro Ala Asn Gly Phe
                   565                     570                     575
   Ile Asn Gly Lys Tyr Asn Tyr Ile Val Glu Thr Pro Met Ile Ala Asp
               580                     585                     590
   Thr Leu Arg Ser Gly Leu Asp Ile Ser Thr Leu Leu Ala Ala Asn Thr
           595                     600                     605
   Val His Asp Ala Pro Ser Ala Ser His Leu Pro Phe Asp Ile Asn Asp
       610                     615                     620
   Pro Ala Val Leu Asn Thr Leu His His Leu Met Leu His Met Arg Ser
   625                     630                     635                     640
   Ile Tyr Pro Ile Asn Asp Ser Ser Lys Asn Ala Thr Gly Ile Ala Leu
                   645                     650                     655
   Gly Arg Tyr Pro Glu Asp Val Tyr Asp Gly Tyr Gly Val Gly Glu Gly
               660                     665                     670
   Asn Pro Trp Val Leu Ala Thr Cys Ala Ala Ser Thr Thr Leu Tyr Gln
           675                     680                     685
   Leu Ile Tyr Arg His Ile Ser Glu Gln His Asp Leu Val Val Pro Met
       690                     695                     700
   Asn Asn Asp Cys Ser Asn Ala Phe Trp Ser Glu Leu Val Phe Ser Asn
   705                     710                     715                     720
   Leu Thr Thr Leu Gly Asn Asp Glu Gly Tyr Leu Ile Leu Glu Phe Asn
                   725                 730                 735
   Thr Pro Ala Phe Asn Gln Thr Ile Gln Lys Ile Phe Gln Leu Ala Asp
               740                 745                 750
   Ser Phe Leu Val Lys Leu Lys Ala His Val Gly Thr Asp Gly Glu Leu
           755                 760                 765
   Ser Glu Gln Phe Asn Lys Tyr Thr Gly Phe Met Gln Gly Ala Gln His
       770                 775                 780
   Leu Thr Trp Ser Tyr Thr Ser Phe Trp Asp Ala Tyr Gln Ile Arg Gln
   785                 790                 795                 800
   Glu Val Leu Gln Ser Leu
                   805
(2) INFORMATION FOR SEQ ID NO:12:
   (i) SEQUENCE CHARACTERISTICS:
      (A) LENGTH: 54 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: N
   (iv) ANTI-SENSE: N
   (ix) FEATURE
      (A) NAME/KEY: -
      (B) LOCATION: 1..54
      (D) OTHER INFORMATION: /label= linker /note= "linker used to create pAYE274"
   (xi) SEQUENCE DESCRIPTION: SEQ ID NO:12:
   ACGGCCCCCC CGGCCACAAA CACAAATATT GATAATATAA AGATGAAGTG GGTA          54
(2) INFORMATION FOR SEQ ID NO:13:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: DNA (genomic)
   (ix) FEATURE
      (A) NAME/KEY: -
      (B) LOCATION: 1..60
      (D) OTHER INFORMATION: /label= Linker /note= "Synthetic oligonucleotide linker used to construct pAYE309"
   (xi) SEQUENCE DESCRIPTION: SEQ ID NO:13:
   AGCTTTATTT CCCTTCTTTT TCTCTTTAGC TCGGCTTATT CCAGGAGCTT GGATAAAAGA        60
(2) INFORMATION FOR SEQ ID NO:14:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: N
   (iv) ANTI-SENSE: N
   (ix) FEATURE:
      (A) NAME/KEY: misc_feature
      (B) LOCATION: 1..19
      (D) OTHER INFORMATION: /function= "Linker" /note= "Linker used in construction of pDXL200. Contains Sfil, Bglll and Hindlll sites."
   (xi) SEQUENCE DESCRIPTION: SEQ ID NO:14:
   GTACGGCCCC CCCGGCCAGA TCTAAGCTT                                           29